(19) Europäisches Patentamt
European Patent Office
Office européen des brevets

(11) **EP 3 876 257 A1**

(12) **EUROPEAN PATENT APPLICATION**
published in accordance with Art. 153(4) EPC

(43) Date of publication:
**08.09.2021 Bulletin 2021/36**

(51) Int Cl.:
*H01H 35/00* (2006.01)     *A61B 5/026* (2006.01)
*A61B 5/0285* (2006.01)     *H01L 31/12* (2006.01)

(21) Application number: **19880898.2**

(22) Date of filing: **30.10.2019**

(86) International application number:
**PCT/JP2019/042568**

(87) International publication number:
**WO 2020/090883 (07.05.2020 Gazette 2020/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.10.2018   JP 2018203998
29.11.2018   JP 2018223552
29.11.2018   JP 2018223473**

(71) Applicant: **Kyocera Corporation
Kyoto-shi, Kyoto 612-8501 (JP)**

(72) Inventor: **ITAKURA, Yoshiaki
Kyoto-shi, Kyoto 612-8501 (JP)**

(74) Representative: **Viering, Jentschura & Partner
mbB
Patent- und Rechtsanwälte
Am Brauhaus 8
01099 Dresden (DE)**

(54) **OPTICAL SENSOR DEVICE**

(57)     An optical sensor device includes a substrate, a light-receiving element, a light-emitting element, a first transparent substrate, and a second transparent substrate. The substrate includes a first opening, and a second opening at a distance from the first opening. The light-receiving element is in the first opening. The light-emitting element is in the second opening, and at a distance from the light-receiving element. The first transparent substrate is placed on an upper surface of the substrate and bonded to the substrate to close the first opening and the second opening. The second transparent substrate is placed on an upper surface of the first transparent substrate.

F I G.  1

**Description**

Technical Field

The present disclosure relates to an optical sensor device.

Background Art

Optical sensor devices including measurement sensors that can easily and quickly measure bio-information such as the bloodstream or a state of fluid flowing through, for example, a semiconductor device have been sought. For example, the bloodstream can be measured using the optical Doppler effect. When the blood is irradiated with light, the light is scattered at blood cells such as red blood cells. A speed of travel of the blood cells is calculated from a frequency of the irradiated light and a frequency of the scattered light. For example, Japanese Patent Application Laid-Open No. 2011-134463 (Patent Document 1) discloses such an optical sensor device that can measure the bloodstream, etc.

However, in the optical sensor device disclosed in Patent Document 1 intended for, for example, the blood, when the light emitted from a light-emitting element hits at least two objects, two light beams are reflected back. One of the target objects is the blood vessel, and the other is the blood. Here, when a light-receiving element detects these two reflected light beams, it is probable that the intensity of the light beam reflected off the blood vessel is increased and the intensity of the light beam reflected off the blood that is a measurement target is decreased.

Summary

An optical sensor device according to an embodiment of the present disclosure includes a substrate, a light-receiving element, a light-emitting element, a first transparent substrate, and a second transparent substrate. The substrate includes a first opening, and a second opening at a distance from the first opening. The light-receiving element is in the first opening. The light-emitting element is in the second opening, and at a distance from the light-receiving element. The first transparent substrate is placed on an upper surface of the substrate and bonded to the substrate to close the first opening and the second opening. The second transparent substrate is placed on an upper surface of the first transparent substrate.

Brief Description of Drawings

FIG. 1 illustrates a perspective view showing an optical sensor device according to a first embodiment of the present disclosure.
FIG. 2 illustrates an exploded perspective view showing the optical sensor device according to the first embodiment of the present disclosure.
FIG. 3 illustrates a cross-sectional view showing the optical sensor device according to the first embodiment of the present disclosure.
FIG. 4 illustrates a cross-sectional view showing parameters of the optical sensor device according to the first embodiment of the present disclosure.
FIG. 5 illustrates a cross-sectional view showing an optical sensor device according to another embodiment relevant to the first embodiment of the present disclosure.
FIG. 6 illustrates a cross-sectional view showing the optical sensor device according to the other embodiment relevant to the first embodiment of the present disclosure.
FIG. 7 illustrates a cross-sectional view showing the optical sensor device according to the other embodiment relevant to the first embodiment of the present disclosure.
FIG. 8 illustrates a cross-sectional view showing the optical sensor device according to the other embodiment relevant to the first embodiment of the present disclosure.
FIG. 9 illustrates a cross-sectional view showing the optical sensor device according to the other embodiment relevant to the first embodiment of the present disclosure.
FIG. 10 illustrates a cross-sectional view showing the optical sensor device according to the other embodiment relevant to the first embodiment of the present disclosure.
FIG. 11 illustrates a perspective view schematically showing an optical sensor device according to second and third embodiments of the present disclosure.
FIG. 12 illustrates an exploded perspective view schematically showing the optical sensor device according to the second and third embodiments of the present disclosure.

FIG. 13 illustrates a cross-sectional view showing the optical sensor device according to the second embodiment of the present disclosure.

FIG. 14 illustrates a cross-sectional view showing parameters of the optical sensor device according to another embodiment relevant to the second embodiment of the present disclosure.

FIG. 15 illustrates a plan view showing the optical sensor device according to the second embodiment of the present disclosure.

FIG. 16 illustrates a plan view showing the optical sensor device according to the second embodiment of the present disclosure.

FIG. 17 illustrates a cross-sectional view showing the optical sensor device according to the third embodiment of the present disclosure.

FIG. 18 illustrates a cross-sectional view showing the optical sensor device according to another embodiment relevant to the third embodiment of the present disclosure.

FIG. 19 illustrates a cross-sectional view showing the optical sensor device according to the other embodiment relevant to the third embodiment of the present disclosure.

FIG. 20 illustrates a cross-sectional view showing the optical sensor device according to the other embodiment relevant to the third embodiment of the present disclosure.

FIG. 21 illustrates a plan view showing the optical sensor device according to the third embodiment of the present disclosure.

FIG. 22 illustrates a plan view showing the optical sensor device according to the third embodiment of the present disclosure.

Description of Embodiments

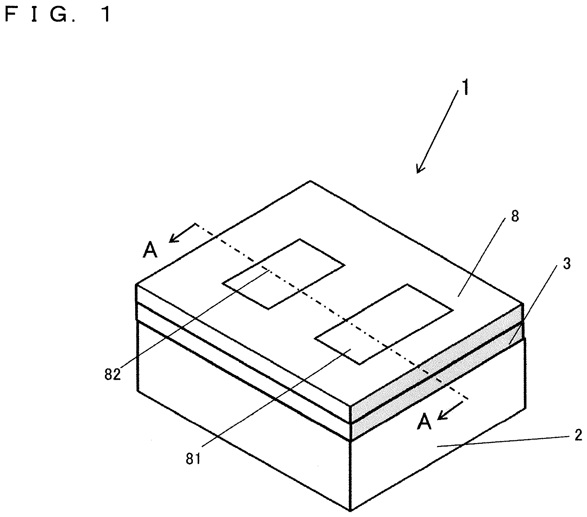

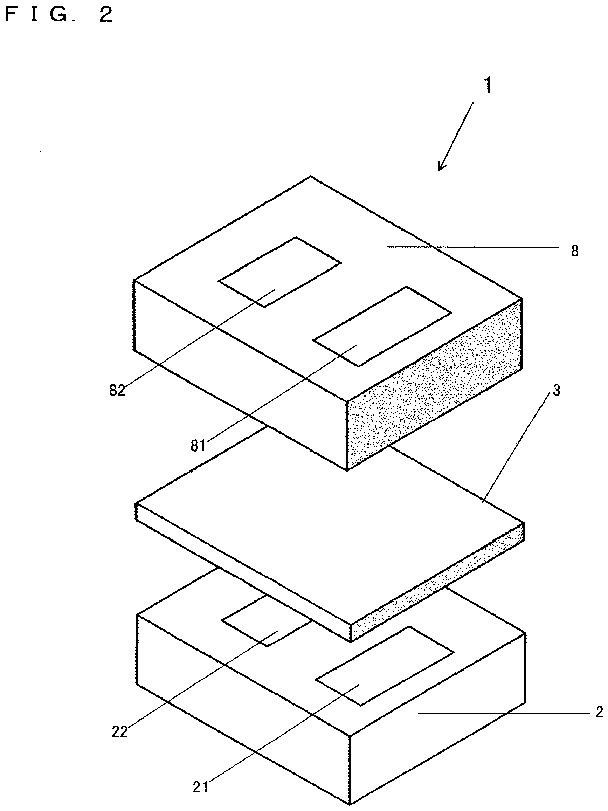

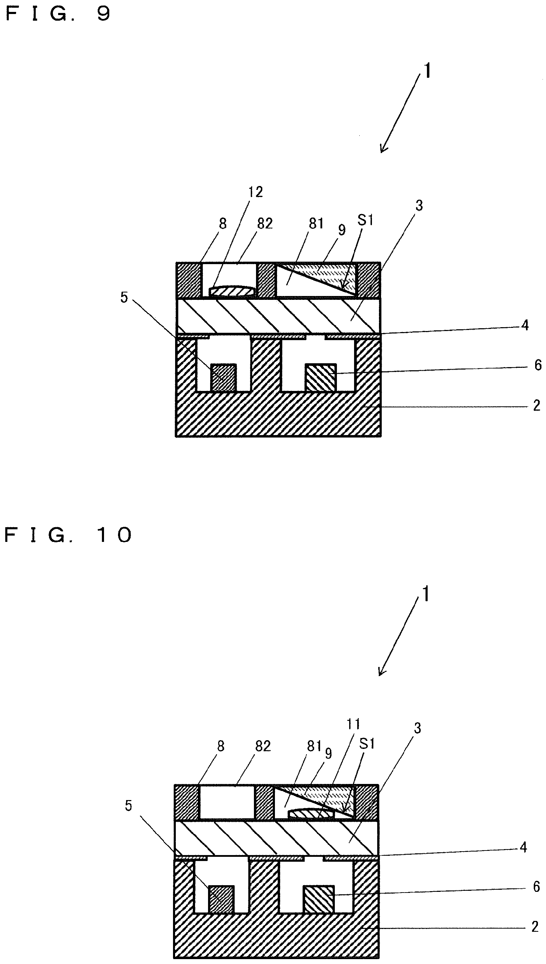

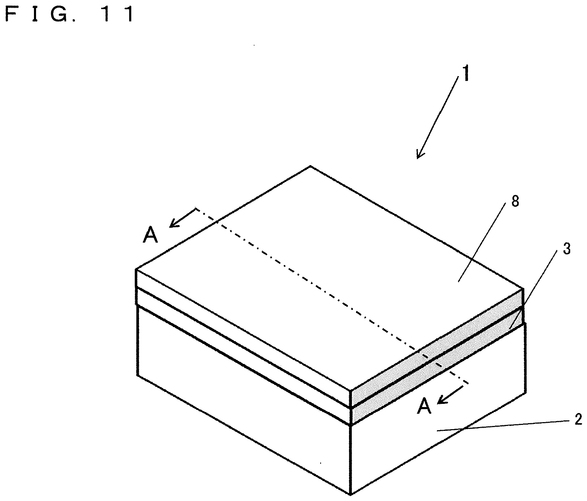

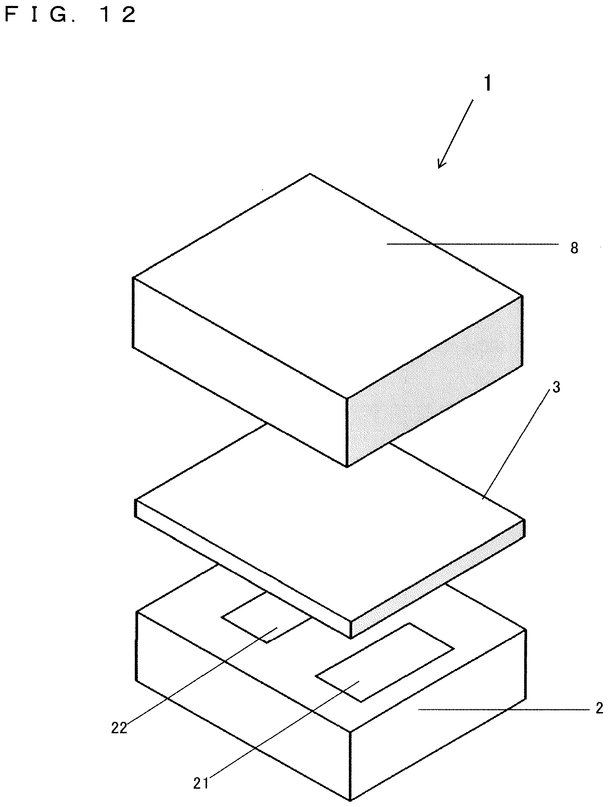

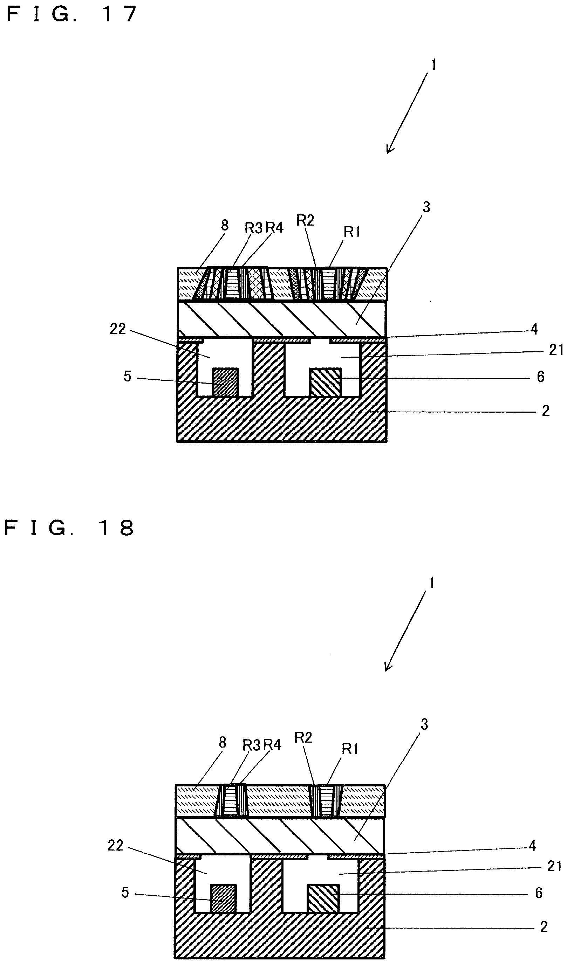

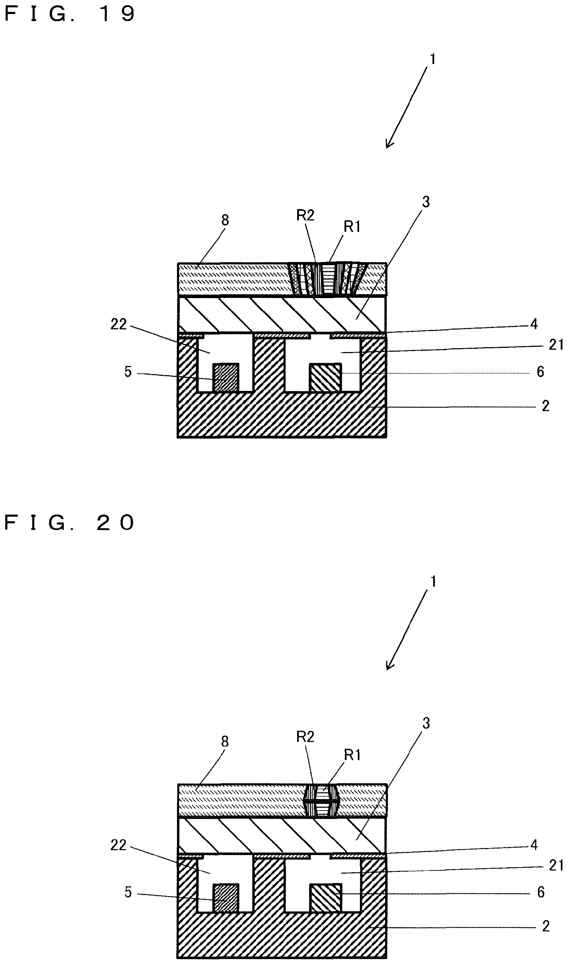

In FIGS. 1 to 22, an optical sensor device 1 includes a substrate 2, a first transparent substrate 3, a light shielding film 4, a light-emitting element 5, a light-receiving element 6, and a second transparent substrate 8.

The substrate 2 may be rectangular in a plan view. The substrate 2 may be formed by laminating a plurality of dielectric layers. For example, the dimensions of the substrate 2 range from 0.5 mm to 5 mm, and its thickness ranges from 0.5 mm to 5 mm in a plan view. The dielectric layers of the substrate 2 may be made of, for example, a ceramic material or an organic material.

When the substrate 2 is a wiring substrate made of a ceramic material (a ceramic wiring substrate), conductors such as connection pads, interconnections, and signal traces are formed in the dielectric layers made of the ceramic material.

Examples of the ceramic material contained in the ceramic wiring substrate include sintered aluminum oxide, sintered mullite, sintered silicon carbide, sintered aluminum nitride, sintered silicon nitride, and sintered glass ceramic.

When the substrate 2 is a wiring substrate made of an organic material (an organic wiring substrate), a wiring conductor such as signal traces to be described later is formed in an insulating layer made of the organic material. The organic wiring substrate is formed from a plurality of organic dielectric layers. The organic wiring substrate may be any as long as it includes dielectric layers made of an organic material, such as a printed wiring substrate, a build-up wiring substrate, or a flexible wiring substrate. Examples of the organic material contained in the organic wiring substrate include an epoxy resin, a polyimide resin, a polyester resin, an acryl resin, a phenolic resin, and a fluorine resin.

This substrate 2 includes at least two recesses as openings. One of the two recesses is a first opening 21 for accommodating the light-receiving element 6, and the other of the two recesses is a second opening 22 for accommodating the light-emitting element 5. The first opening 21 and the second opening 22 are formed to be opened on the same principal surface 21 of the substrate 2 (the first surface of the substrate 2).

The optical sensor device 1 according to an embodiment of the present disclosure is preferably used as a measurement sensor that measures the flow of fluid such as the bloodstream, with the optical Doppler effect. The measurement sensor includes the light-emitting element 5 that irradiates an object to be measured with light, and the light-receiving element 6 that receives the light scattered by the object to be measured to take advantage of the optical Doppler effect. Particularly, when measuring the bloodstream, the measurement sensor irradiates a part of the body, for example, a finger with light from the outside. Then, the measurement sensor receives the light scattered by the blood cells contained in the blood that flows through the blood vessel under the skin, and measures the bloodstream from changes in the frequency. Thus, in the optical sensor device 1, the light-emitting element 5 and the light-receiving element 6 are disposed at a predetermined distance, based on the position relationship between the irradiated light and the scattered light. The first opening 21 and the second opening 22 are formed according to the position relationship between the light-emitting element 5 and the light-receiving element 6.

Appropriately setting the dimensions of the first opening 21 and the second opening 22 according to the dimensions of the light-emitting element 5 and the light-receiving element 6, respectively, to be accommodated therein will suffice. The dimensions of each of the light-emitting element 5 and the light-receiving element 6 range, for example, from 0.1 mm × 0.1 mm × 0.1 mm to 1.5 mm × 1.5 mm × 1.2 mm. For example, when a vertical-cavity surface-emitting laser (VCSEL) is used as the light-emitting element 5, the second opening 22 may be, for example, rectangular or square. For example, the second opening 22 has a vertical dimension ranging from 0.3 mm to 2.0 mm, a horizontal dimension ranging from 0.3 mm to 2.0 mm, and a depth ranging from 0.3 mm to 1.0 mm. When a plane-of-incidence photodiode is used as a light emitting diode (LED) or the light-receiving element 6, the first opening 21 may be, for example, rectangular or square. For example, the first opening 21 has a vertical dimension ranging from 0.3 mm to 2.0 mm, a horizontal dimension ranging from 0.3 mm to 2.0 mm, and a depth ranging from 0.4 mm to 1.5 mm. The first opening 21 and the second opening 22 (the light-emitting element 5 and the light-receiving element 6) may be spaced apart to the extent that the light emitted by the light-emitting element 5 does not directly enter the light-receiving element 6. Furthermore, forming a lightproof wall between the first opening 21 and the second opening 22 (between the light-emitting element 5 and the light-receiving element 6) can shorten a distance between the first opening 21 and the second opening 22 (between the light-emitting element 5 and the light-receiving element 6). For example, the center of the first opening 21 may be placed to overlap the center of the light-receiving element 6, and the center of the second opening 22 may be placed to overlap the center of the light-emitting element 5 in planar perspective.

The first opening 21 and the second opening 22 may be, for example, circular, square, or rectangular, or of another shape. Each of the first opening 21 and the second opening 22 may have a uniform cross-section parallel to the principal surface of the substrate 2 in the depth direction. Alternatively, each of the first opening 21 and the second opening 22 may be a stepped recess having a uniform cross-section identical to that of its opening up to a predetermined depth and a uniform cross-section smaller than the former one from the predetermined depth to the bottom. When the first opening 21 and the second opening 22 are stepped recesses similarly to this embodiment of the present disclosure, the bottom of the first opening 21 includes a region for mounting the light-receiving element 6, and the bottom of the second opening 22 includes a region for mounting the light-emitting element 5. Furthermore, a connection pad electrically connected to the light-emitting element 5 or the light-receiving element 6 is placed on the stepped surface.

Furthermore, the substrate 2 may include signal traces which are electrically connected to the light-emitting element 5 or the light-receiving element 6, through which an electrical signal is transmitted to the light-emitting element 5, and through which an electrical signal is output from the light-receiving element 6. The signal traces may include a bonding wire that is a connection part to be connected to the light-emitting element 5 or the light-receiving element 6, a connection pad to which the bonding wire is connected, a via conductor electrically connected to the connection pad and extending from immediately below the connection pad to the lower surface of the substrate 2 (the second surface of the substrate 2), and an external connection terminal electrically connected to the via conductor. The external connection terminal is disposed on the lower surface of the substrate 2, and is electrically connected to a connection terminal of an external mounting substrate on which the measurement sensor including the optical sensor device 1 is mounted, with a terminal connection material such as solder.

For example, in the external connection, a nickel layer 0.5 $\mu$m to 10 $\mu$m thick and a metal layer 0.5 $\mu$m to 5 $\mu$m thick may be deposited in turn by the plating technique for increasing the wettability with a bonding material such as solder and increasing the corrosion resistance.

The first transparent substrate 3 covers the upper surface of the substrate 2 (the first surface of the substrate 2), and is bonded to the first surface of the substrate 2 through the bonding material. The first transparent substrate 3 closes and seals the first opening 21 and the second opening 22 accommodating the light-receiving element 6 and the light-emitting element 5, respectively. The first transparent substrate 3 is a plate-shaped component made of an insulating material, and the first transparent substrate 3 may be composed of a material having optical transparency allowing the light emitted from the light-emitting element 5 accommodated in the second opening 22 to pass through the first transparent substrate 3 and allowing the light received by the light-receiving element 6 accommodated in the first opening 21 to pass through the first transparent substrate 3.

Semiconductor laser elements such as the VCSEL can be used as the light-emitting element 5, and various photodiodes including a silicon photodiode, a GaAs photodiode, an InGaAs photodiode, and a germanium photodiode can be used as the light-receiving element 6. Appropriately selecting the light-emitting element 5 and the light-receiving element 6 depending on, for example, the type of an object to be measured or the type of a parameter to be measured will suffice.

When the bloodstream is measured, for example, using the optical Doppler effect, the VCSEL functioning as the light-emitting element 5 may be the one that can emit laser light with a wavelength of 850 nm. When another measurement is performed, selecting the light-emitting element 5 that emits laser light with a wavelength that fits the measurement purpose will suffice. The light-receiving element 6 may be any as long as it can receive the light emitted from the light-emitting element 5 in the absence of change in the wavelength of the light. If the wavelength of the light is changed, the light-receiving element 6 may be the one that can receive the light with the changed wavelength.

Although the light-emitting element 5, the light-receiving element 6 and the connection pad are electrically connected to the connection pad by, for example, a bonding wire 32 in this embodiment, another connection method such as flip chip bonding, bump connections, or connection using an anisotropic conductive film may be used.

Furthermore, the first transparent substrate 3 needs to allow the irradiated light to the object to be measured and the scattered light through. Since the characteristics of the irradiated light and the scattered light are determined by a light-emitting element to be mounted, the first transparent substrate 3 may be structured to allow at least the light emitted from the light-emitting element to be mounted through. The first transparent substrate 3 is composed of the insulating material whose transmittance of the light with the wavelength that is emitted from the light-emitting element is higher than or equal to 70%, preferably, higher than or equal to 90% will suffice.

Examples of the insulating material of the first transparent substrate 3 can include a transparent ceramic material such as sapphire, a glass material, and a resin material. Examples of the glass material can include borosilicate glass, crystallized glass, quartz, and soda glass. Examples of the resin material can include a polycarbonate resin, an unsaturated polyester resin, and an epoxy resin. The first transparent substrate 3 is, for example, rectangular in a plan view. The dimensions of the first transparent substrate 3 range from 0.5 mm × 1 mm to 5 mm × 5 mm. Furthermore, the first transparent substrate 3 is 0.5 mm to 5 mm thick.

The bonding material bonds the substrate 2 to the first transparent substrate 3. Specifically, the bonding material bonds an upper surface of the substrate 2 to a lower surface of the first transparent substrate 3 at the periphery. The bonding material is ring-shaped on the upper surface of the substrate 2, and is a sealant for maintaining the airtightness and the water-tightness in the first opening 21 and the second opening 22 of the substrate 2. Since the light-receiving element 6 and the light-emitting element 5 accommodated in the first opening 21 and the second opening 22, respectively, are susceptible to, for example, moisture, the bonding material is shaped into a ring without a seam to reduce the entry of external moisture.

Furthermore, the bonding material may be lightproof. This lightproof effect of the bonding material can reduce external light entering the first opening 21 and the second opening 22 through the substrate 2 and the first transparent substrate 3.

The lightproof effect of the bonding material may be achieved by absorption of light. Although the lightproof effect may be achieved by reflection of light from the viewpoint of reducing the entry of external light, the stray light generated inside the measurement sensor may be reflected off the bonding material and further received by the light-receiving element. If the bonding material is the one that absorbs the light, the bonding material can absorb the external light to reduce the entry of the external light, and can also absorb the stray light generated inside.

The bonding material functioning as a material with the lightproof effect through absorption of light is obtained by, for example, dispersing a light absorbing material into a resin adhesive with capability of bonding the substrate 2 and the first transparent substrate 3, such as an epoxy resin or a conductive silicon resin. Examples of the light absorbing material can include inorganic pigments. Examples of the inorganic pigments can include a carbon-based pigment such as carbon black, a nitride-based pigment such as titanium black, and a metal oxide-based pigment such as Cr-Fe-Co system, Cu-Co-Mn system, Fe-Co-Mn system, or Fe-Co-Ni-Cr system. Furthermore, the bonding material may be made of a metal material such as solder. Examples of the metal material can include brazing filler metals such as Sn-Ag, Sn-Ag-Cu, Au-Sn, Au-Sn-Ag, and Au-Si.

The light shielding film 4 may be placed on the lower surface of the first transparent substrate 3. The light shielding film 4 is formed by, for example, vapor-depositing, sputtering, or baking a metal material, for example, a metal, e.g., Cr, Ti, Al, Cu, Co, Ag, Au, Pd, Pt, Ru, Sn, Ta, Fe, In, Ni, or W, or an alloy of some of these metals. The light shielding film 4 is, for example, 50 nm to 400 nm thick. The light shielding film 4 is placed to overlap the light-emitting element 5. The overlapping herein indicates a state where the light shielding film 4 covers a part of the light-emitting element 5. The light shielding film 4 partially includes a through hole through which at least the light emitted by the light-emitting element 5 and the reflected light that reaches the light-receiving element 6 pass. The light shielding film 4 may be placed on the upper surface of the second transparent substrate 8 to be described later. This can reduce the incidence of unnecessary scattered light onto the light-receiving element 6.

In the light shielding film 4, a first through hole 81 is, for example, circular or rectangular, and the dimensions range from Φ 50 μm to Φ 1 mm. Furthermore, a second through hole 82 is, for example, circular or rectangular, and the dimensions range from Φ 5 μm to Φ 500 μm. The second transparent substrate 8 according to the first embodiment of the present disclosure includes the first through hole 81 in a position overlapping the first opening 21. Furthermore, the second transparent substrate 8 includes the second through hole 82 in a position overlapping the second opening 22 and at a distance from the first through hole 81. A lens 9 is placed in the first through hole 81.

The second transparent substrate 8 is placed on the upper surface of the first transparent substrate 3. The second transparent substrate 8 can be made of, for example, a transparent ceramic material such as sapphire, a glass material, or a resin material. Examples of the glass material can include borosilicate glass, crystallized glass, quartz, and soda glass. Examples of the resin material can include a polycarbonate resin, an unsaturated polyester resin, and an epoxy resin. The dimensions of the second transparent substrate 8 may be the same as those of the first transparent substrate 3 in a plan view. The thickness of the second transparent substrate 8 may be any with consideration given to a distance to a target object.

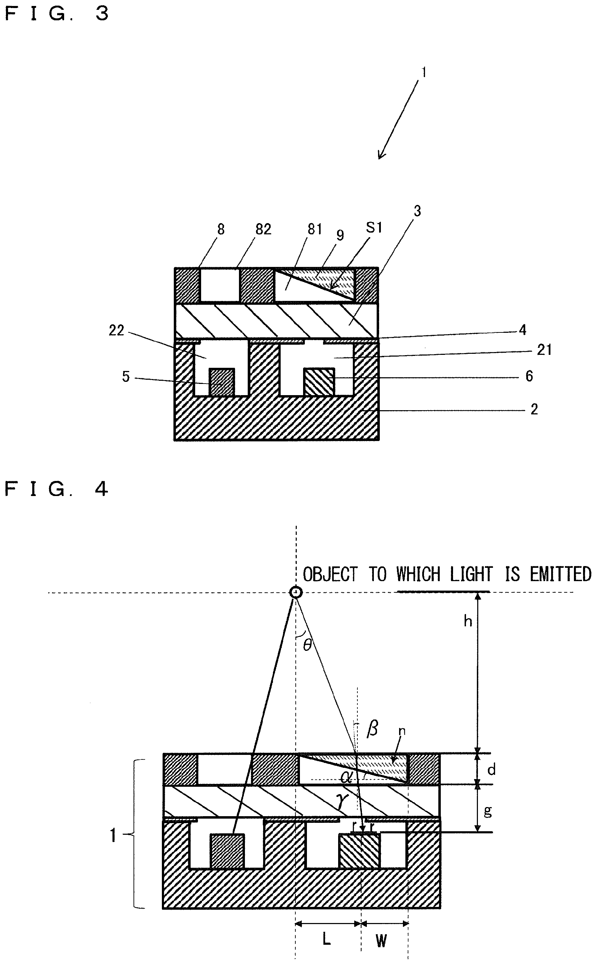

The lens 9 has a first sloped surface S1. In a cross-sectional view, the first sloped surface S1 is sloped downward from the light-emitting element 5 side to the light-receiving element 6 side (sloped from the left side to the right side in FIG. 3). In other words, the first sloped surface S1 is more sloped toward the first transparent substrate 3 as it comes closer from the light-emitting element 5 side to the light-receiving element 6 side in a cross-sectional view. Specifically, the lens is thicker from the inside of the substrate 2 toward the outside of the substrate 2. The cross-sectional view herein means a cross-section in a vertical direction and a direction of connecting the centers of the light-emitting element 5 and the light-receiving element 6 in the optical device 1.

The optical sensor device 1 according to the embodiment of the present disclosure includes the lens 9 with the first sloped surface S1. Thus, the light reflected off the surface of a target object (reference light) is easily totally reflected, and more intense light reflected off the target object (measured light) can easily enter the light-receiving element 6.

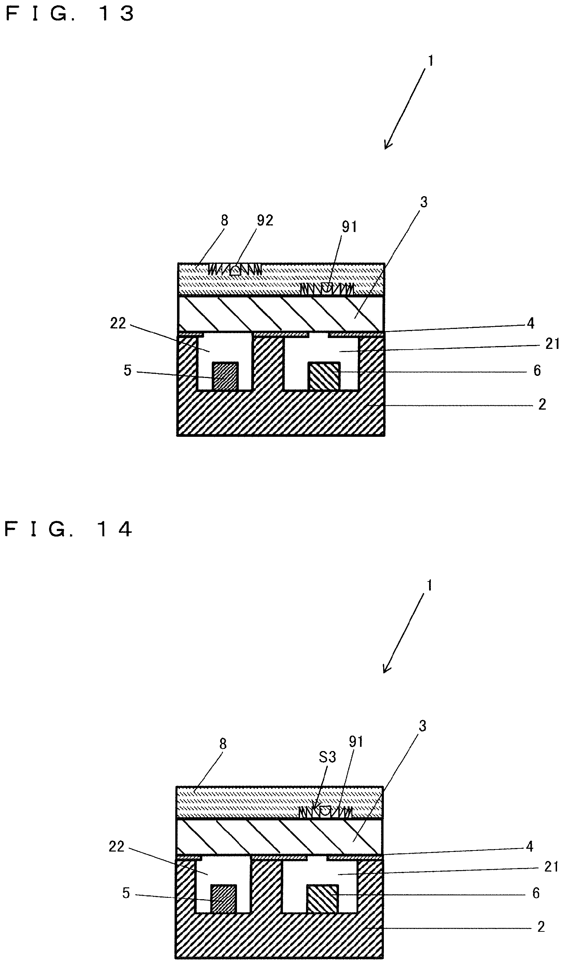

According to the second embodiment of the present disclosure, a lower surface of the second transparent substrate 8 includes a first diffractive lens 91 whose surface is convex and concave in a position overlapping the first opening 21 in a plan view as illustrated in FIGS. 13 and 14. The concave portions and the convex portions may be alternately and concentrically arranged from the center of the first diffractive lens 91, particularly, from the position overlapping the light-receiving element 6 in a plan view toward the outside. Furthermore, the convex portions of the first diffractive lens 91 may have third sloped surfaces S3 toward the center. The slope angles of the third sloped surfaces S3 may be gradually increased with respect to a direction perpendicular to the thickness direction of the second transparent substrate 8. Here, the slope angles are angles of the slopes with respect to the plane such as the upper surface and the lower surface of the second transparent substrate 8. Specifically, only the convex portions, only the concave portions, or the convex portions and the concave portions may be alternately and concentrically arranged from the center toward the outside in a plan view. The spaces between the convex portions and the concave portions may be narrower toward the outside. The first diffractive lens 91 with such a concentric arrangement can be, for example, a spherical lens. The first diffractive lens 91 can be produced by micromachining the surface. The third sloped surfaces S3 of sloped convex portions can reduce the incidence of the light reflected off an object other than a target object and the reflected light that has an angle of reflection larger than that of the target object onto the light-receiving element 6. Alternatively, the third sloped surfaces S3 enable the light to be easily totally reflected. Furthermore, the concentric arrangement can reduce the incidence of the reflected light that has an angle of reflection larger than that of the target object onto the light-receiving element 6 in any direction, or enables the light to be easily totally reflected. The reflected light means the light emitted from the light-emitting element 5 and reflected off a target object or a certain object other than the target object.

Furthermore, the upper surface of the second transparent substrate 8 may include a second diffractive lens 92 whose surface is convex and concave in a position overlapping the second opening 22 in a plan view. Similarly to the first diffractive lens 91, the convex portions of the second diffractive lens 92 may have fourth sloped surfaces S4 toward the center. Only the convex portions, only the concave portions, or the convex portions and the concave portions may be alternately and concentrically arranged from the center, particularly, from the position overlapping the light-emitting element 5 in a plan view toward the outside. Furthermore, the slope angles of the fourth sloped surfaces S4 may be increased with respect to the thickness direction of the second transparent substrate 8 and the vertical direction. Specifically, only the convex portions, only the concave portions, or the convex portions and the concave portions may be alternately and concentrically arranged from the center toward the outside in a plan view. The spaces between the convex portions and the concave portions may be narrower toward the outside. The second diffractive lens 92 with such a concentric arrangement can be, for example, a spherical lens. The second diffractive lens 92 can be produced by micromachining the surface. The sloped convex portions can focus the light by the fourth sloped surfaces S4, and allow the light from the light-emitting element 5 to concentratedly hit a target object. Furthermore, the concentric arrangement allows the light incident from the light-emitting element 5 in any direction to be easily focused.

The optical sensor device 1 according to the embodiment of the present disclosure includes the first diffractive lens 91 in the position overlapping the light-receiving element 6. This can totally reflect the light including the reference light except the desired light to be measured. Alternatively, this can keep the position at which the light including the reference light except the desired light to be measured is focused from overlapping the light-receiving element 6. Consequently, the magnitude of signal light incident on the light-receiving element 6 can be relatively increased.

According to the invention of the third embodiment of the present disclosure, the second transparent substrate 8 includes a first region R1 and a second region R2 with different refractive indexes, in positions overlapping the first opening 21 in a plan view. A plurality of regions including the first region R1 and the second region R2 involve the plane and the thickness direction. In a plan view, the first region R1 and the second region R2 may be concentrically arranged in order from the center of the plurality of regions, particularly, from the positions overlapping the light-receiving element 6 toward the outside. Here, a region with a different refractive index may be placed outside the first region R1 and the second region R2. Furthermore, regions with different refractive indexes may be concentrically arranged in positions overlapping the first opening 21 in a plan view. In a cross-sectional view in a vertical direction of the optical sensor device 1 and a direction of connecting the centers of the light-emitting element 5 and the light-receiving element 6, the boundary between the first region R1 and the second region R2 may be sloped so that each of the first region R1 and the second region R2 has a lower surface smaller than an upper surface, and in the second transparent substrate 8, a boundary of the second region R2 opposite to the boundary between the first region R1 and the second region R2 may have a slope angle larger than that of the boundary between the first region R1 and the second region R2. The second transparent substrate 8 may include the plurality of regions with different refractive indexes, and the plurality of regions may be concentrically arranged. Specifically, the plurality of regions may be concentrically arranged from the center of the plurality of regions toward the outside in a plan view with respect to the lower surface of the second transparent substrate 8. The widths of the regions may be narrower toward the outside. For example, a spherical lens may be bonded for use as the second transparent substrate 8 with the concentric arrangement. Furthermore, the second transparent substrate 8 can be produced by micromachining, for example, changing a refractive index using laser light. The concentric arrangement can reduce the incidence of the reflected light that has an angle of reflection larger than that of the target object onto the light-receiving element 6 in any direction, or enables the light to be easily totally reflected.

Furthermore, the second transparent substrate 8 includes a third region R3 and a fourth region R4 with different refractive indexes, in positions overlapping the second opening 22 in a plan view. The third region R3 and the fourth region R4 may be concentrically arranged in order from the center of the plurality of regions, particularly, from the position overlapping the light-emitting element 5 toward the outside in a plan view. Furthermore, the boundary between the third region R3 and the fourth region R4 may be sloped so that each of the third region R3 and the fourth region R4 has a lower surface larger than an upper surface, and in the second transparent substrate 8, a boundary of the fourth region R4 opposite to the boundary between the third region R3 and the fourth region R4 may have a slope angle larger than that of the boundary between the third region R3 and the fourth region R4. Specifically, the plurality of regions may be concentrically arranged from the center of the regions toward the outside in a plan view with respect to the upper surface of the second transparent substrate 8. The widths of the regions may be narrower toward the outside. For example, a spherical lens may be bonded for use as the second transparent substrate 8 with the concentric arrangement. Furthermore, the second transparent substrate 8 can be produced by micromachining, for example, changing a refractive index using laser light. The concentric arrangement allows the light incident from the light-emitting element 5 in any direction to be easily focused.

The optical sensor device 1 according to the embodiment of the present disclosure includes the first region R1 and the second region R2 with different refractive indexes, in the positions overlapping the light-receiving element 6. This can totally reflect the light including the reference light except the desired light to be measured. Alternatively, this can keep the position at which the light including the reference light except the desired light to be measured is focused from overlapping the light-receiving element 6. Consequently, the magnitude of the light incident on the light-receiving element 6 can be relatively increased.

[Method for manufacturing optical sensor device]

A method for manufacturing the optical sensor device 1 will be described. First, the substrate 2 is produced in a method for manufacturing a multilayer wiring substrate. When the substrate 2 is a ceramic wiring substrate and is made of alumina as its ceramic material, first, admixing feedstock powder such as alumina ($Al_2O_3$), silica ($SiO_2$), calcia (CaO), or magnesia (MgO) with an appropriate organic solvent or an appropriate solvent produces slurry. This slurry is molded into a sheet by a known method such as doctor-blading or calendar roll to obtain a ceramic green sheet (hereinafter may be referred to as a green sheet). Then, the green sheet is die cut into a predetermined shape. Furthermore, admixing feedstock powder such as tungsten (W) or a glass material with an organic solvent or a solvent produces a metal paste. This metal paste is pattern printed on the surface of the green sheet in a printing method such as screen printing. A via conductor is produced by forming a through hole on the green sheet and filling the through hole with the metal paste by, for example, the screen printing. A metallized layer to be, for example, a ground conductor is formed on the top surface using the metal paste. Laminating a plurality of the green sheets obtained in such a manner, and simultaneously firing the green sheets at a temperature approximately 1600°C produce the substrate 2.

On the other hand, the first transparent substrate 3 is prepared by cutting a glass material into a predetermined shape through, for example, trimming or cutting-off. The light shielding film 4 to be described later is formed on the lower surface of the first transparent substrate 3 through, for example, vapor-deposition, sputtering, or baking.

Although the via conductors are aligned in the vertical direction in the substrate 2, the via conductors need not be aligned but may be formed in the substrate 2 with displacements, for example, through inner layer wiring or using an inner ground conductor layer as long as they are electrically connected from the upper surface of the substrate 2 to the external connection terminal on the lower surface.

For example, machining or an imprint technique using a die may be applied to the second transparent substrate 8, similarly to the method for manufacturing the first transparent substrate 3 and the lens 9, etc. Examples of the machining include a method for directly processing a rectangular parallelepiped transparent substrate, and a method for embedding a lens or a sloped glass separately produced into a pierced base substrate and fixing the lens or the sloped glass to the base substrate using, for example, an adhesive.

[Another embodiment relevant to the first embodiment of the optical sensor device]

In the optical sensor device 1 according to the other embodiment of the present disclosure, the first sloped surface S1 of the lower surface of the lens 9 may have a slope angle at which the reflected light larger than the light reflected off a target object is totally reflected. This structure can reduce the incidence of light other than a target object, and increase the intensity of the light reflected off the target object. Thus, more accurate measurement can be performed.

Specifically, assuming that $\theta 1$ denotes an angle of reflection at which the light emitted from the light-emitting element 5 hits fluid, for example, the blood as a target object and is reflected off, and $\theta 2$ denotes an angle of reflection at which the light hits an object covering the fluid as the target object, for example, the surface of the blood vessel and is reflected off, $\alpha 2 \leq \alpha < \alpha 1$ may hold under the following conditions:

$$\text{Condition 1:} \quad \alpha \geq \sin^{-1}(n1/n2) - \sin^{-1}(n1/n2*\sin\theta 2) = \alpha 2;$$

and

$$\text{Condition 2:} \quad \alpha < \sin^{-1}(n1/n2) - \sin^{-1}(n1/n2*\sin\theta 1) = \alpha 1.$$

$\alpha$ is the one illustrated in FIG. 4.

Furthermore, the refractive index of the lens 9 may be larger than that of air. If this is applied to the equation above, n1 is the refractive index of air, and n2 is the refractive index of the lens 9. Increase in the refractive index of n2 can cause the total reflection of light with ease.

Furthermore, parameters that can increase the intensity of light desired to be measured are described below using the reference numerals in FIG. 4. Here, h denotes a distance from an object for reflection to the upper surface of the lens 9 with the first sloped surface S1, d denotes the thickness of the second transparent substrate 8, g denotes a distance from the lens 9 with the first sloped surface S1 to the light receiver, r denotes a half of the width of the light receiver, L denotes a horizontal distance from the object for reflection to the center of the light receiver, W denotes a horizontal distance from the right end of the lens 9 with the first sloped surface S1 to a light receiving point, n denotes the refractive index of the lens 9 with the first sloped surface S1, and $\theta$, $\beta$, and $\gamma$ denote angles. Here, when the light is received at a position at the distance L under a condition of $\alpha > 0°$, W = ((L - htan$\theta$ - dtan$\beta$)(1 - tan$\alpha$tan$\gamma$) - gtan$\gamma$ (1 - tan$\alpha$tan$\beta$))/(tan$\alpha$ (tan$\gamma$-tan$\beta$)) holds. At the left end and the right end, W1 = ((L $\pm$ r - htan$\theta$ - dtan$\beta$)(1-tan$\alpha$tan$\gamma$) - gtan$\gamma$ (1 - tan$\alpha$tan$\beta$))/(tan$\alpha$ (tan$\gamma$ - tan$\beta$)) holds.

Furthermore, the upper surface of the lens 9 may be sloped from the light-emitting element 5 side to the light-receiving element 6 side in a cross-sectional view. In other words, the upper surface of the lens 9 may include a second sloped surface S2. Here, the second sloped surface S2 is sloped upward. This enables the light to be more easily totally reflected.

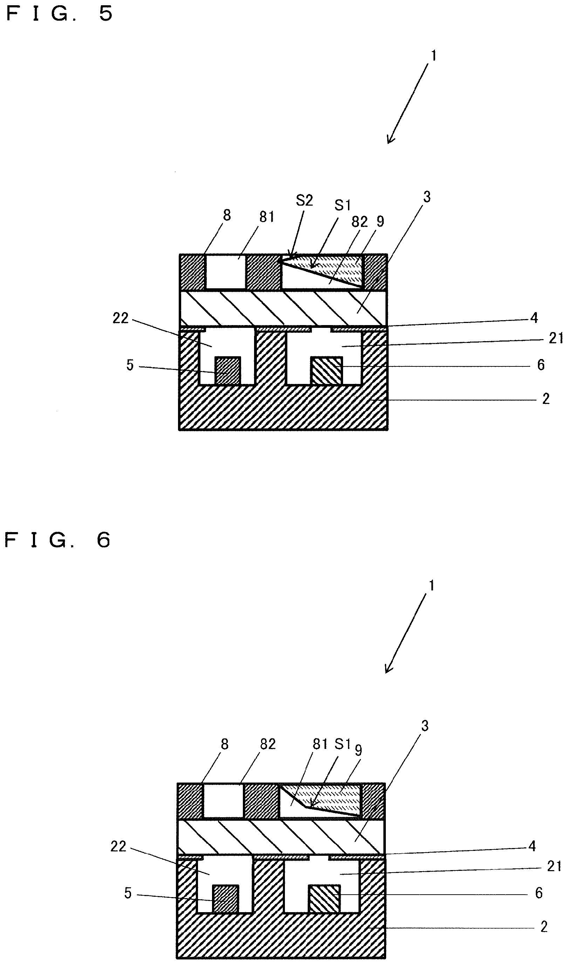

Here, only a part of the upper surface of the lens 9 closer to the light-emitting element 5 as illustrated in FIG. 5 or the entirety of the upper surface may be sloped, so that only the reference light is easily totally reflected.

Similarly, only a part of the lower surface of the lens 9 closer to the light-emitting element 5 or the entirety of the lower surface may be sloped, so that only the reference light is easily totally reflected.

Furthermore, the lens 9 may be sloped symmetrically in the vertical direction. Here, the upper surface and the lower surface of the lens 9 may be partly sloped.

[Another embodiment relevant to the first embodiment of the optical sensor device]

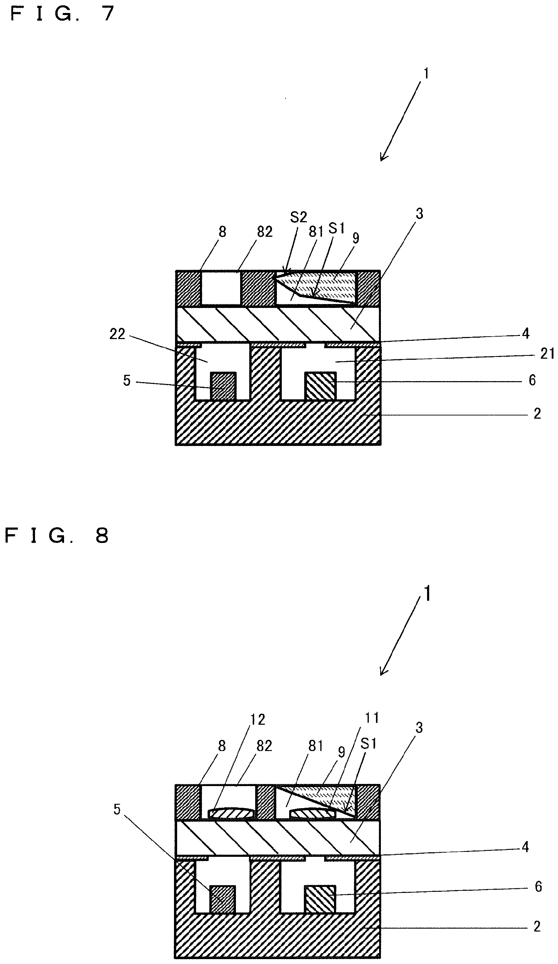

As illustrated in FIG. 8 , a first condenser lens 11 may be attached to the upper surface of the first transparent substrate 3 below the lens 9, in a position overlapping the first opening 21 and the first through hole 81 in the optical sensor device 1 according to the other embodiment of the present disclosure. For example, the dimensions of the first condenser lens 11 range from $\Phi$ 20 $\mu$m to $\Phi$ 2 mm, and its thickness ranges from 0.5 mm to 2 mm in a plan view. Furthermore, the first condenser lens 11 is made of, for example, a glass material such as quartz glass or borosilicate glass, or a resin material such as acryl, polycarbonate, styrene, or polyolefine. The first condenser lens 11 may have optical transparency allowing the light emitted from the light-emitting element 5 to pass through the light-receiving element 6. The first condenser lens 11 may have the properties of refracting, in an optical axis direction, light through, for example, a convex lens with light focusing properties. The first condenser lens 11 can improve the light focusing properties toward the light-receiving element 6 by refracting the diffused light that is the light irradiated from the light-emitting element 5 to make converging rays or collimated light.

Here, a second condenser lens 12 may be further attached to the upper surface of the first transparent substrate 3 in a position overlapping the second opening 22 and the second through hole 82. For example, the dimensions of the second condenser lens 12 range from $\Phi$ 70 $\mu$m to $\Phi$ 2 mm, and its thickness ranges from 50 $\mu$m to 2 mm in a plan view. Furthermore, the second condenser lens 12 is made of, for example, a glass material such as quartz glass or borosilicate glass, or a resin material such as acryl, polycarbonate, styrene, or polyolefine. The second condenser lens 12 may have optical transparency allowing the light irradiated from the light-emitting element 5 through. Furthermore, the second condenser lens 12 may have the properties of refracting, in an optical axis direction, light through, for example, a convex lens with light focusing properties. The second condenser lens 12 can improve the light focusing properties by refracting the diffused light that is the light irradiated from the light-emitting element 5 to make converging rays or collimated light.

The optical sensor device 1 is mounted on an external mounting substrate and used. For example, a control element that controls an emission of the light-emitting element 5, and an arithmetic element that calculates the blood flow velocity, etc., from an output signal of the light-receiving element 6 are mounted on the external mounting substrate.

In measurement, the optical sensor device 1 receives the current for controlling the light-emitting element from the external mounting substrate through the external connection terminal, with the fingertip of the finger being in contact with the surface of the second transparent substrate 8 as an object to be measured (target object). Then, the light-emitting element 5 receives the current through, for example, the via conductors and the connection pad, and emits the light for measurement. When the irradiated light reaches the fingertip through the first transparent substrate 3, the light is scattered at blood cells in the blood. Upon receipt of the scattered light through the first transparent substrate 3, the light-receiving element 6 outputs an electrical signal corresponding to the amount of the received light. The output signal passes through the connection pad and the via conductors, and is sent from the optical sensor device 1 to the external mounting substrate through the external connection terminal.

In the external mounting substrate, the arithmetic element receives the signal output from the optical sensor device 1. For example, the arithmetic element can calculate the blood flow velocity by analyzing the intensity of the scattered light received by the light-receiving element 6 for each frequency.

In the optical sensor device 1 according to the other embodiment of the present disclosure, the lower surface of the first transparent substrate 3 may be spaced from the substrate 2 between the first opening 21 and the second opening 22. Specifically, the substrate 2 includes a lightproof wall between the first opening 21 and the second opening 22, and a part of the upper end of the wall is missing. Since this enables the reference light to directly reach the light-receiving element 6, more accurate measurement can be performed.

[Another embodiment relevant to the second embodiment of the optical sensor device]

The optical sensor device 1 according to the other embodiment of the present disclosure may include the first diffractive lens 91 that totally reflects the reflected light with an angle of incidence larger than that of the light reflected off a target object. Alternatively, the optical sensor device 1 may include the first diffractive lens 91 that can keep the position at which the reflected light with an angle of incidence larger than that of the light reflected off at least a target object is focused from overlapping the light-receiving element 6. This structure can reduce the incidence of the light reflected off an object other than the target object onto the light-receiving element 6, and relatively increase the intensity of the light reflected off the target object. Thus, more accurate measurement can be performed.

Furthermore, the first diffractive lens 91 and the second diffractive lens 92 may be of the same shape. The shapes and the positions of the lenses may be the same on the upper surface and the lower surface as illustrated in FIG. 13. This facilitates the processing. The first diffractive lens 91 and the second diffractive lens 92 may be of different shapes. Here, the lenses may have the same dimensions, or may be of the same shape and geometrically similar.

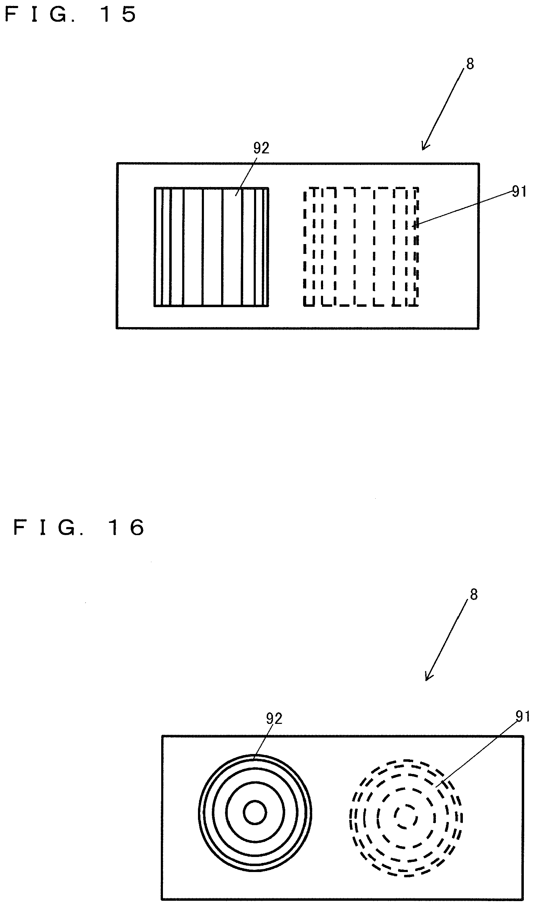

The convex portions and the concave portions of the first diffractive lens 91 may be alternately arranged from the center of the first diffractive lens 91 toward the outside. Specifically, in a plan view as illustrated in FIG. 15, the outer edge of the first diffractive lens 91 may be rectangular, and convex and concave portions may be formed to be parallel in one side of the rectangular outer edge. Similarly, the slope angles of the third sloped surfaces S3 of the convex portions may be increased as they are more distant from a position directly above the light-receiving element 6. Specifically, intervals between the concave portions, between the convex portions, or between the concave portions and the convex portions are narrower toward the outside of the first diffractive lens 91 with respect to the center of the first diffractive lens 91 in a plan view. This can reduce the incidence of the reflected light that has an angle of reflection larger than that of the target object onto the light-receiving element 6 in any direction.

Similarly to the first diffractive lens 91, the second diffractive lens 92 may also include convex and concave portions alternately arranged from the center toward the outside. Specifically, in a plan view, the outer edge of the second diffractive lens 92 may be rectangular, and convex and concave portions may be formed to be parallel in one side of the rectangle. Similarly, the slope angles of the third sloped surfaces S3 of the convex and concave portions may be increased as they are more distant from a position directly above the light-emitting element 5. Specifically, the widths of the convex and concave portions are narrower from the center toward the outside in a plan view.

[Another embodiment relevant to the second embodiment of the optical sensor device]

The optical sensor device 1 according to the other embodiment of the present disclosure is advantageous because the reflected light with an angle of incidence larger than that of the light reflected off a target object can be totally reflected. Alternatively, the position at which reflected light with an angle of incidence larger than that of reflected light from at least a target object is focused can be kept from overlapping the light-receiving element 6. Here, the optical sensor device 1 may include the first region R1 and the second region R2 with different refractive indexes. This structure can reduce the incidence of the light other than the target object onto the light receiver, and relatively increase the intensity of the light reflected off the target object. Thus, more accurate measurement can be performed.

Furthermore, the first region R1 and the third region R3, and the second region R2 and the fourth region R4 may be of the same shape, and symmetrically arranged. This facilitates the processing. Furthermore, the first region R1 and the third region R3, and the second region R2 and the fourth region R4 may be of different shapes.

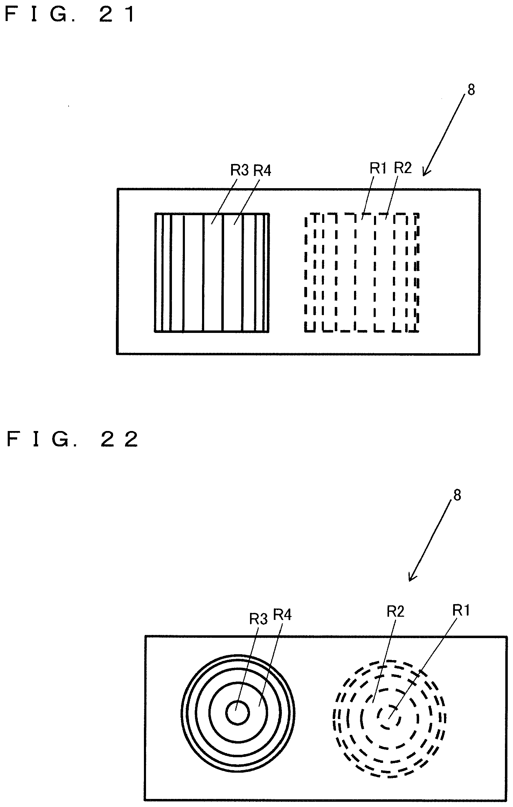

Furthermore, the first region R1 and the second region R2 may be aligned from the center toward the outside. Specifically, in a plan view as illustrated in FIG. 21, the outer edge of the first region R1 and the second region R2 may be rectangular, and the first region R1 and the second region R2 may be aligned in parallel in one side of the rectangle. Furthermore, the third region R3 and the fourth region R4 may be aligned from the center toward the outside.

Furthermore, the refractive index of the second region R2 may be smaller than that of the first region R1. Here, the light is easily totally reflected off a boundary surface between the first region R1 and the second region R2. Furthermore, the light incident on the light-receiving element 6 can be easily reduced.

The present disclosure is not limited by the examples of the embodiments. Various modifications of values, etc., are possible. Furthermore, the method for mounting the elements in the embodiments is not specified. The embodiments according to the present disclosure can be combined without departing from the scope. Although application of the optical sensor device in the embodiments according to the present disclosure to a pulse oximeter is described, it is applicable to other devices operated by a pair of sensor elements of a light-emitting element and a light-receiving element, for example, an integrated proximity/ambient light sensor device, a proximity sensor device, and a distance measuring sensor device.

Explanation of Reference Signs

| | |
|---|---|
| 1 | optical sensor device |
| 2 | substrate |
| 3 | first transparent substrate |
| 4 | light shielding film |
| 5 | light-emitting element |
| 6 | light-receiving element |
| 8 | second transparent substrate |
| 9 | lens |
| 21 | first opening |
| 22 | second opening |
| 81 | first through hole |
| 82 | second through hole |
| 11 | first condenser lens |
| 12 | second condenser lens |
| 91 | first diffractive lens |
| 92 | second diffractive lens |
| R1 | first region |
| R2 | second region |
| R3 | third region |
| R4 | fourth region |
| S1 | first sloped surface |
| S2 | second sloped surface |
| S3 | third sloped surface |

**Claims**

1.  An optical sensor device, comprising:

    a substrate including a first opening, and a second opening at a distance from the first opening;
    a light-receiving element in the first opening;
    a light-emitting element in the second opening;
    a first transparent substrate placed on an upper surface of the substrate and bonded to the substrate to close the first opening and the second opening; and
    a second transparent substrate placed on an upper surface of the first transparent substrate.

2.  The optical sensor device according to claim 1,

    wherein the second transparent substrate includes a first through hole in a position overlapping the first opening in a plan view, and a second through hole in a position overlapping the second opening in a plan view and at a distance from the first through hole, and
    a lens is placed in the first through hole, and a lower surface of the lens includes a first sloped surface sloped downward from the light-emitting element side to the light-receiving element side in a cross-sectional view.

3.  The optical sensor device according to claim 1,
    wherein a lower surface of the second transparent substrate includes a first diffractive lens whose surface is convex and concave in a position overlapping the first opening in a plan view.

4.  The optical sensor device according to claim 1,
    wherein the second transparent substrate includes a first region and a second region with different refractive indexes, in positions overlapping the first opening in a plan view.

5.  The optical sensor device according to claim 2,
    wherein the first sloped surface has a slope angle at which reflected light that has an angle of reflection larger than an angle of reflection of light reflected off a target object is totally reflected.

6.  The optical sensor device according to claim 2 or 5,
    wherein a refractive index of the lens is larger than a refractive index of air.

7.  The optical sensor device according to one of claims 2, 5, and 6,
    wherein an upper surface of the lens includes a second sloped surface in a cross-sectional view, the second sloped surface being sloped upward from the light-emitting element side to the light-receiving element side.

8.  The optical sensor device according to one of claims 2 and 5 to 7, comprising or in which the following is placed at least one of a first condenser lens placed in the first through hole and below the lens, and a second condenser lens placed in the second through hole.

9.  The optical sensor device according to claim 3,
    wherein an upper surface of the second transparent substrate includes a second diffractive lens whose surface is convex and concave in a position overlapping the second opening in a plan view.

10. The optical sensor device according to claim 3 or 9,

    wherein the first diffractive lens includes concave portions and convex portions, and
    the convex portions have third sloped surfaces toward a center of the first diffractive lens, the third sloped surfaces having slope angles increased from the center toward an outside of the first diffractive lens with respect to a direction perpendicular to the lower surface of the second transparent substrate.

11. The optical sensor device according to claim 9 or 10,
    wherein the first diffractive lens and the second diffractive lens are of a same shape.

12. The optical sensor device according to one of claims 3 and 9 to 11,
    wherein the concave portions and the convex portions of the first diffractive lens are alternately and concentrically arranged.

13. The optical sensor device according to one of claims 3 and 9 to 12,
wherein the concave portions and the convex portions of the first diffractive lens are alternately and concentrically arranged from the center of the first diffractive lens toward an outside of the first diffractive lens.

14. The optical sensor device according to claim 4,
wherein the second transparent substrate includes a third region and a fourth region with different refractive indexes, in positions overlapping the second opening in a plan view.

15. The optical sensor device according to claim 4 or 14,

wherein the first region is placed to overlap a center of the light-receiving element in a plan view, and
the refractive index of the first region is larger than the refractive index of the second region.

16. The optical sensor device according to claim 14 or 15,
wherein the refractive index of the third region is identical to the refractive index of the first region, and the refractive index of the fourth region is identical to the refractive index of the second region.

17. The optical sensor device according to one of claims 4 and 14 to 16,
wherein the second transparent substrate includes a plurality of regions with different refractive indexes in positions overlapping the first opening in a plan view, the plurality of regions being concentrically arranged in a plan view.

18. The optical sensor device according to one of claims 4 and 14 to 17,
wherein the first region and the second region are sloped so that each of the first region and the second region has a lower surface smaller than an upper surface.

19. The optical sensor device according to one of claims 4 and 14 to 18,
wherein each of the first region and the second region has a shape symmetric about a center of a thickness of the second transparent substrate.

20. The optical sensor device according to one of claims 4 and 14 to 19,
wherein a refractive index of the second transparent substrate decreases from a center of a plurality of regions toward an outside.

21. The optical sensor device according to one of claims 1 to 20, further comprising
a light shielding film placed on an upper surface of the second transparent substrate.

F I G. 1

FIG. 2

F I G. 3

F I G. 4

OBJECT TO WHICH LIGHT IS EMITTED

F I G. 5

F I G. 6

FIG. 7

FIG. 8

FIG. 9

FIG. 10

FIG. 11

FIG. 12

1

8

3

2

22

21

F I G. 1 3

F I G. 1 4

FIG. 15

FIG. 16

F I G. 1 7

F I G. 1 8

FIG. 19

FIG. 20

FIG. 21

FIG. 22

## INTERNATIONAL SEARCH REPORT

| International application No. |
|---|
| PCT/JP2019/042568 |

A. CLASSIFICATION OF SUBJECT MATTER
Int.Cl.  H01H35/00(2006.01)i, A61B5/026(2006.01)i, A61B5/0285(2006.01)i,
           H01L31/12(2006.01)i

According to International Patent Classification (IPC) or to both national classification and IPC

B.  FIELDS SEARCHED

Minimum documentation searched (classification system followed by classification symbols)
Int.Cl. H01H35/00, A61B5/026, A61B5/0285, H01L31/12

Documentation searched other than minimum documentation to the extent that such documents are included in the fields searched
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y<br>A | WO 2017/110291 A1 (KYOCERA CORP.) 29 June 2017, paragraphs [0036]–[0037], [0040], [0054], fig. 6 & US 2018/0310836 A1, paragraphs [0044]–[0045], [0048], [0062], fig. 6 & EP 3395242 A1 & CN 108135516 A & KR 10-2018-0053383 A | 1, 3, 9–13, 21<br>2, 4–8, 14–20 |
| Y<br>A | JP 3-15129 A (OMRON CORP.) 23 January 1991, page 2, lower right column, lines 16–19, page 4, upper left column, lines 5–17, fig. 5, 7 & US 5130531 A, column 6, lines 30–34, column 8, lines 22–39, fig. 5, 7 | 1, 3, 9–13, 21<br>2, 4–8, 14–20 |

☒ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 December 2019 (17.12.2019) | 07 January 2020 (07.01.2020) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

| International application No. |
|---|
| PCT/JP2019/042568 |

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2008/065699 A1 (PIONEER CORP.) 05 June 2008, paragraph [0045], fig. 2 & US 2010/0056887 A1, paragraph [0071], fig. 2 & EP 2087837 A1 | 21 |
| A | GB 2486000 A (STMICROELECTRONICS (RESEARCH & DEVELOPMENT) LIMITED) 06 June 2012 & US 2012/0133956 A1 | 2, 4-8, 14-20 |
| A | JP 2007-225923 A (HAMAMATSU PHOTONICS K.K.) 06 September 2007 & US 2009/0202251 A1 & WO 2007/097240 A1 & EP 1993148 A1 & KR 10-2008-0100332 A & CN 101371373 A | 2, 4-8, 14-20 |
| A | JP 2003-207578 A (TOTO KIKI KABUSHIKI KAISHA) 25 July 2003 (Family: none) | 2, 5-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011134463 A